# EUROPEAN PATENT SPECIFICATION

(11) **EP 1 685 827 B1**
(45) Date of publication and mention of the grant of the patent: **27.10.2010**
(21) Application number: 06250328.9
(22) Date of filing: 21.01.2006
(51) Int. Cl.: A61K 8/81, A61Q 5/00, A61Q 5/06

(54) **Use of rheology modifiers for aqueous systems in hair gels**
Verwendung von Rheologiemodifizierern für wässrige Systeme in Haargelen
Utilisation de modificateurs rhéologiques pour des systèmes aqueux pour des gels appliqués aux cheveux

(30) Priority: 31.01.2005 EP 05290212
(43) Date of publication of application: 02.08.2006
(73) Proprietor: Rohm and Haas Company, Philadelphia, PA 19106-2399 (US)
(72) Inventor: Collin, Jennifer Reichl, Devon, Pennsylvania 19333 (US); Keenan, Andrea Claudette, Plymouth Meeting Pennsylvania 19462 (US); Lee, Wai Kin Albert, Singapore 738081 (SG); Reeve, Paul Francis David, 06130 Grasse (FR); Zeng, Fanwen, Belle Mead New Jersey 08502 (US)
(74) Representative: Kent, Venetia Katherine

(56) References cited:
- EP-A- 0 329 419
- EP-A- 1 402 877
- WO-A-98/06757
- US-A- 4 828 752
- US-A- 5 057 241
- US-A- 5 587 154

## Description

This invention relates to the use in a hair gel of an aqueous system comprising rheology-modifying polymers.

Rheology modifiers are used in aqueous cleaning products, such as, for example, shampoo, to increase viscosity, to suspend particles, or both. In cases where suspending particles are important, the rheology modifier especially is useful to increase viscosity at low shear rates while maintaining flow properties of the product at higher shear rates. In addition, rheology modifiers provide effective, heat-age stable suspensions of particulate material or beads dispersed in an aqueous phase. A variety of copolymer rheology modifiers made from vinyl monomers have been used for this purpose. For example, U.S. Patent Application Pub. No. 2004/0063855, discloses an acrylic emulsion copolymer of methacrylic acid, an alkyl acrylate, acrylic acid and stearyloxypoly(ethyleneoxy)₂₀ethyl methacrylate useful for suspending particulates. Despite the many various known rheology modifiers, there is still a need for a rheology modifier which combines good stability, favorable rheological properties, and which also provides compositions which exhibit smooth flow and high efficiency with suspension of particulates, air bubbles, silicones, and similar materials.

EP-A-1402877 discloses aqueous compositions for high surfactant systems at a pH between 5 and 13 which comprise crosslinked copolymers. The crosslinkers can be diallyl phthalate or divinylbenzene.

US-A-5057241 discloses aqueous compositions at a pH of 4 - 10 for preparing detergent compositions which comprise crosslinked copolymers. The crosslinker is divinylbenzene.

The present invention uses an aqueous composition having a pH of from 4-10 and comprising from 0.1% to 8% of one or more crosslinked copolymers, wherein each of the one or more crosslinked copolymers independently comprises from 2.5 to 35 weight percent (meth)acrylic acid residues, from 10 to 80 weight percent C₂-C₄ alkyl (meth)acrylate residues, from 2 to 25 weight percent lipophilically modified (meth)acrylate residues, and from 0.001 to 7.5 weight percent residues of a crosslinker wherein the crosslinker has no ester or amide functionality.

Unless otherwise specified, percentages are weight percentages based on the entire composition, polymer, or copolymer, as the case may be. As used herein the term "(meth)acrylic" refers to acrylic or methacrylic, and "(meth)acrylate" refers to acrylate or methacrylate or mixtures thereof. The term "acrylic polymers" refers to polymers comprising one or more (meth)acrylic monomers, such as, for example, acrylic acid ("AA"), methacrylic acid ("MAA") and their esters, and copolymers comprising at least 50% (meth)acrylic monomers. Esters of AA and MAA include, for example, methyl methacrylate ("MMA"), ethyl methacrylate ("EMA"), butyl methacrylate ("BMA"), hydroxyethyl methacrylate ("HEMA"), methyl acrylate ("MA"), ethyl acrylate ("EA"), butyl acrylate ("BA"), ethylhexyl acrylate ("EHA"), and hydroxyethyl acrylate ("HEA"), as well as other alkyl esters of AA or MAA, including the lipophilically modified monomers described below. Preferably, acrylic polymers have at least 75% of monomer residues derived from (meth)acrylic acid or (meth)acrylate monomers, or both, more preferably at least 90%, even more preferably at least 95%, and most preferably at least 98%. The term "vinyl monomer" refers to a monomer suitable for addition polymerization and containing a single polymerizable carbon-carbon double bond. As used herein, the term "residue" means a monomer unit within a polymer. Unless otherwise specified, the terms "the copolymer" or "the crosslinked copolymer" refer independently to each of the one or more crosslinked copolymers in the aqueous composition of this invention.

The copolymers used according to the invention contain lipophilically-modified (meth)acrylate residues each of which may contain either one, or a plurality of, lipophilic groups. According to one embodiment of this invention, such groups are suitably in the same copolymer component as and attached to hydrophilic chains, such as for example polyoxyethylene chains. According to another embodiment, the copolymer may contain a vinyl group which may be used to copolymerize the polymer to other vinyl-containing entities to alter or improve the properties of the polymer. Alternatively other copolymerization systems may be used. The polymerizable group may be attached to the lipophilic group directly, or indirectly, for example via one or more, for example up to 60, preferably up to 40, water-soluble linker groups, such as, for example, -CH[R]CH₂O or -CH[R]CH₂NH- groups wherein R is hydrogen or methyl. Alternatively, the polymerizable group may be attached to the lipophilic group by reaction of the hydrophilic component, such as, for example polyoxyethylene, with a urethane compound containing unsaturation. The molecular weight of the lipophilic-modifying group or groups is preferably selected together with the number of such groups to give the required minimum lipophilic content in the copolymer, and preferably, for satisfactory performance in a wide range of systems.

The amount of lipophilically-modified component in the copolymers useful in the present invention preferably is at least 5%, more preferably at least 10%, and most preferably at least 16%; and preferably is no more than 20%.

The lipophilic-modifying groups themselves are preferably straight chain saturated alkyl groups, but may be aralkyl or alkyl carbocyclic groups such as, for example, alkylphenyl groups, having at least 6, and up to 40 carbon atoms, although branched chain groups are also useful in this invention. It is understood that the alkyl groups may be either of synthetic or of natural origin and, in the latter case particularly, may contain a range of chain lengths. For example, naturally sourced stearic acid, even of commercially pure quality may contain only about 90% of stearic chains, up to about 7% of palmitic chains and a proportion of other chains and lower quality products may contain substantially less stearic acid. It is intended herein that reference to the chain length of such groups is to the predominant chain length which is present as more than 50%, preferably in more than 75%, of the chains.

It is an important subsidiary feature of the invention that the chain length of the lipophilic-modifying groups be minimized. When the chain is primarily alkyl groups, the predominant chain length preferably is below 40, more preferably from 8 to 22, and most preferably from 10 to 18 carbon atoms. The hydrophilic component of the lipophilically-modified copolymer may suitably be a polyoxyethylene component preferably comprising at least one chain of at least 2, preferably at least 5, more preferably at least 10, and up to 60, preferably up to 40, more preferably up to 30 ethylene oxide units. Such components are usually produced in a mixture of chain lengths.

Preferably, the C₂-C₄ alkyl (meth)acrylate residues in the copolymer used in this invention are C₂-C₃ alkyl (meth)acrylate residues, and most preferably EA. Preferably, the amount of C₂-C₄ alkyl (meth)acrylate residues is at least 20%, more preferably at least 30%, even more preferably at least 40% and most preferably at least 50%. Preferably, the amount of C₂-C₄ alkyl (meth)acrylate residues is no more than 70%, more preferably no more than 65%, and most preferably no more than 60%. Preferably, the amount of (meth)acrylic acid residues in the copolymer used in the present invention is at least 5%, more preferably at least 7.5%, even more preferably at least 10%, and most preferably at least 15%. Preferably, the amount of (meth)acrylic acid residues is no more than 27.5%, more preferably no more than 25%, and most preferably no more than 22%. (Meth)acrylic acid residues are introduced into the copolymer by inclusion of either (meth)acrylic acid, or a (meth)acrylic acid oligomer having a polymerizable vinyl group, in the monomer mixture used to produce the copolymer. Preferably, the copolymer contains residues derived from (meth)acrylic acid in an amount that provides a total acrylic acid plus methacrylic acid content of at least 15%, more preferably at least 17.5%, and most preferably at least 20%. Preferably, the total acrylic acid plus methacrylic acid content of the copolymer is no more than 65%, more preferably no more than 50%, and most preferably no more than 40%.

Optionally, the copolymer may also contain from 2% to 25%, preferably from 5% to 20%, of a hydrophilic comonomer, preferably one having hydroxyl, carboxylic acid or sulfonic acid functionality. Examples of such hydrophilic comonomers include, for example, 2-hydroxyethyl (meth)acrylate , itaconic acid, and acrylamido-2-methylpropanesulfonic acid.

The copolymers of the present invention are crosslinked, that is, a crosslinker, such as a monomer having two or more ethylenic unsaturated groups, is included with the copolymer components during polymerization. The crosslinker does not have ester or amide functionality. Crosslinking monomers include, for example, divinylbenzene, trimethylolpropane diallyl ether, tetraallyl pentaerythritol, triallyl pentaerythritol, triallyl cyanurate, bis-phenol A diallyl ether, diallyl pentaerythritol and allyl sucroses. Divinylbenzene, trimethylolpropane diallyl ether ('TMPDE') and tetraallyl pentaerythritol are preferred. The amount of crosslinker residues in the polymer may range from 0.001 % to 7.5% but is typically at least 0.01%, preferably at least 0.1%, based on weight of the copolymer components. Preferably, the amount of crosslinker residues in the polymer is no more than 3.0%, more preferably no more than 2.5%. In one embodiment of the invention in which the crosslinker is difunctional, such as, for example, divinylbenzene, preferably the amount of crosslinker residue in the polymer is at least 0.5%, more preferably at least 1%, and most preferably at least 1.5%. In another embodiment of the invention in which the crosslinking agent is more than difunctional, preferably the amount of crosslinker residue in the polymer is no more than 1.0%, more preferably no more than 0.5%.

In one embodiment of the invention, the copolymer is prepared in the presence of a chain transfer agent when a crosslinking agent is used. Examples of suitable chain transfer agents include, for example, carbon tetrachloride, bromoform, bromotrichloromethane, and compounds having a mercapto group, including, for example, 3-mercaptopropionic acid or long chain alkyl mercaptans, and thioesters such as dodecyl-, octyl-, tetradecyl- or hexadecyl-mercaptans or butyl-, isooctyl- or dodecyl-thioglycolates. When used, the amount of chain transfer agent is typically from 0.01 % to 5%, preferably from 0.1% to 1%, based on weight of the copolymer components. In one embodiment of this invention, the crosslinking agent is used in conjunction with a chain transfer agent. These are typically conflicting operations for polymerization purposes, but in the case of this invention it provides a copolymer which, not only is exceptional in efficiency observed but also shows very high compatibility with hydrophilic surfactants, as manifested by increased product clarity.

The copolymer may be prepared by copolymerizing the monomers using known aqueous emulsion polymerization procedures at an acidic pH, or inverse emulsion polymerization at neutral pH, or precipitation or solution polymerization processes. In such processes any other suitable additives known in the art, such as, for example, a free-radical initiator such as peroxygen or diazo compound and, optionally, chain transfer agents may be used. Suitable peroxygen compounds include, for example, peroxides, hydroperoxides, persulfates or organic peroxides. A suitable quantity of initiator may be 0.01% to 3% by weight of the components of the copolymer. The copolymerization temperature is typically 25° C to 92° C, preferably 60°C to 90°C. Typically, the copolymer is recovered by filtration and the copolymer may, if desired, be provided in dry form by spray drying or coagulation. U.S. Patents 4,384,096, 4,663,385, 4,429,097 and 4,514,552 may be consulted for further general and specific details of suitable copolymerization and recovery techniques, and of suitable monomers and additives. If the lipophilically modified copolymers useful in this invention were not crosslinked, their molecular weight would typically be in the range of from 100,000 to 1 million.

The aqueous compositions of the present invention contain from 0.5% to 8% of one or more of the copolymers. Preferably, the amount of the copolymer in the aqueous composition is at least 0.75%, more preferably at least 1%, and most preferably at least 1.25%. Preferably, the amount of the copolymer in the aqueous composition is no more than 4%, more preferably no more than 3%, and most preferably no more than 2.5%. Preferably, the copolymer is an acrylic polymer. The copolymer, in aqueous dispersion or in the dry form, may be blended into an aqueous system to be thickened followed by a suitable addition of acidic or basic material if required.

The aqueous compositions of the present invention optionally contain up to 40% of one or more surfactants. When present, the surfactant(s) preferably is selected from the groups of anionic surfactants characterized by carboxylate, sulfonate, sulfate, or phosphate solubilizing groups, and nonionic surfactants characterized by amide or hydroxyl groups or ethylene oxide chains. Cationic, amphoteric or zwitterionic surfactants may also or alternatively be used provided that they are compatible with the copolymer and other ingredients of the aqueous composition in the quantity required by the invention. Cationic surfactants characterized by amine or ammonium solubilizing groups, and/or amphoteric surfactants characterized by combinations of anionic and cationic solubilizing groups may be selected. Preferred surfactants for use in the practice of the invention may be selected from the C₈ to C₁₈ fatty acids or their water soluble salts; water soluble sulfates of C₈ to C₁₈ alcohols; sulfonated alkylaryl compounds such as, for example, dodecylbenzene sulfonate, alkylphenoxy polyethoxy ethanols, such as, for example with C₇ to C₁₈ alkyl groups and 9 to 40 or more oxyethylene units; ethylene oxide derivatives of long chain carboxylic acids, such as, for example of lauric, myristic, palmitic or oleic acids; ethylene oxide derivatives of long chain alcohols, such as, for example of lauryl or cetyl alcohols; and alkanolamides and polyglucosides, such as, for example the alkyl polyglucosides. Suitable cationic surfactants may be, for example, lauryl pyridinium chloride, octylbenzyltrimethyl-ammonium chloride, dodecyl trimethylammonium chloride and ethylene oxide condensates of primary fatty acid amines.

The compositions of the present invention include other optional ingredients,such as, for example, salts, one or more additional rheology modifiers (such as, for example, Laponite™ clay, cellulosics, carrageenan, xanthan, PEG-150 distearate, and other acrylic or urethane rheology modifiers), organic or inorganic particles (such as, for example, abrasives, beads, mica, encapsulated oil beads), dispersed liquids, silicones, dispersants, biocides, enzymes, bleach, emollients, oils, fragrances, dyes, UVA and UVB absorbers, infrared absorbers, and thioglycolic acid.

We have discovered that the copolymers of this invention are particularly effective as rheology modifiers in aqueous compositions that require thickening or suspending, clarity, and a smooth flow and spreadability without chunkiness, during the application to the substrate on which the product is intended to be used. The product is applied to hair . Typical rheology modifiers in these compositions either give thickening or suspending but not smooth flow or spreadability on the substrate, or they provide a composition with smooth flow, but without efficient thickening. By thickening, we mean that addition of the polymer to the aqueous composition allows for an increase in the viscosity of the composition. By clarity, we mean that the turbidity of the sample is less than 50 NTU, using specifications in U.S. Environmental Protection Agency method 180.1 (Nephelometric Method). By chunkiness, we mean that the formulation applied to the substrate tends to break up into lumps or rough sheets rather than smoothly flow as a continuous film. By smooth flow, we mean that as the formulation is applied to the substrate, it spreads easily and does not break up. Spreadability refers to how easy it is to form a film of the liquid on a substrate. Suspending refers to the even dispersion of particulate or solid material, liquid material, or air throughout the continuous phase of the formulation. Failure of suspension is marked by phase separation of the dispersed material from the continuous phase under a range of storage temperature conditions.

The aqueous composition of the copolymer is used as a hair gel. Typical components of a hair gel, in addition to the rheology modifier, include a film forming hair fixative agent, and sufficient base to neutralize the hair fixative agent, rheology modifier, or both. Optional additives in a hair gel include fragrance, fragrance solubilizer, conditioners, suspended particles, silicones, plasticizers, preservatives, and solvents, such as, for example, ethanol. In one embodiment, the copolymer can itself serve as both a hair fixative agent and rheology modifier. When used in a hair fixative composition, the composition may contain additional hair fixatives. Suitable additional hair fixatives include, for example, any hair fixative polymers listed under CTFA International Cosmetic Ingredients Dictionary and Handbook, such as, for example, Acrylates Copolymer, PVP, PVP/VA, Acrylamide/Acrylamidomethylpropanesulfonate/Methacrylates Polymer, Polyquaternium-4, Polyquaternium-11,PQ-7, PQ-39, PQ-2, PQ-10, PQ-16, PQ-16, PQ-46, PQ-28, PQ-55, PVP/Dimethylaminoethyl methacrylate copolymer, Guar hydroxypropyl trimonium chloride, Vinyl caprolactam/PVP/Dimethyl aminoethyl methacrylate copolymer, PVP and dimethicone, PQ-28 and dimethicone, PVP/vinylcaprolactam/DMAPA acrylates copolymer, PVP/DMAPA acrylates copolymer, modified corn starch, Acrylates/Hydroxyesters acrylates Copolymer, Acrylamide/Sodium Acryloyldimethyltaurate/Acrylic Acid Copolymer, and Polyvinylcaprolactam.

### EXAMPLES

Compositions of typical polymers useful in the compositions of this invention include, for example, the following:

| Polymer | Composition |
|---|---|
| #1 | 18 Lipo1/52EA/10MAA/20AA//1.6DVB /0.1n-DDM |
| #2 | 18 Lipo1/52EA/10MAA/20AA//2.0DVB/0.1n-DDM |
| #3 | 18 Lipo1/52EA/10MAA/20AA//1.8 DVB/0.1n-DDM |
| #4 | 18 Lipo1/52EA/10MAA/20AA//0.135 triallyl isocyanurate/0.1n-DDM |
| #5 | 18 Lipo1/52EA/10MAA/ 20AA//0.12Tetraallyl Pentaerythritol/0.1n-DDM |
| #6 | 18 Lipo1/52EA/10MAA/ 20AA//0.116Trimethylolpropane Diallyl ether/0.1n-DDM |
| #7 | 3 Lipo3^{b}/15Lipo1/52EA/10MAA/20AA/0.116TMPDE/0.1n-DDM |
| #8 | 6 Lipo3/12 Lipo1/52EA/10MAA/20AA/0.116TMPDE/0.1n-DDM |
| #9 | 9 Lipo3/9 Lipo1/52EA/10MAA/20AA/0.116TMPDE/0.1n-DDM |
| #10 | 18 Lipo1/52EA/10MAA/ 20AA//0.08Tetraallyl Pentaerythritol/0.1n-DDM |

| | |
|---|---|
| a. Lipo1 is a lipophilically modified monomer having a linear saturated C₁₆₋₁₈ alkyl group connected through from 18 to 26 oxyethylene residues to a methacryloyl group. b. Lipo3 is a lipophilically modified monomer having a linear saturated C₂₀₋₂₄ alkyl group connected through 20-28 oxyethylene residues to a methacryloyl group. c. nDDM is n-dodecyl mercaptan. d. TMPDE: trimethylolpropane diallyl ether | |

### Example 1. Alcohol Free Hair Gel with Acrylates/Hydroxyesters Acrylates Copolymer

### Composition:

| **Ingredient** | **Weight Percent in Final Formulation** | **Trade Name (Supplier)** |
|---|---|---|
| Acrylates/Hydroxyesters | 2.0 | Acudyne™ 180 or Acudyne™ DHR hair |
| Acrylates Copolymer | | fixative (Rohm and Haas Company) |
| Aminomethyl Propanol | 0.53 | AMP-95 (Angus) |
| Glycerin | 1.0 | Glycerin (Merck) |
| Panthenol | 0.1 | D-Panthenol USP (Roche) |
| EDTA | 0.05 | Titriplex™ III (Merck) |
| Fragrance | 0.18 | |
| Polysorbate 20 | 0.72 | Crillet™ I (Croda) |
| Methylisothiazolinone | 0.10 | Neolone™ 950 preservative (Rohm and Haas Company) |
| Polymer #6 | 5.0 | |
| Deionized Water | 90.32 | |

### Method of preparation:

1. Predilute Acrylates/Hydroxyesters Acrylates Copolymer with a portion of the total deionized water charge. This is Part 1.
2. Predilute Aminomethyl Propanol with a portion of the total deionized water charge. This is Part 2.
3. Add Part 1 to Part 2 with stirring. The combined mixture is Part 3.
4. Combine fragrance and Polysorbate 20.
5. Add Glycerin, Panthenol, EDTA, Fragrance/Polysorbate 20 mixture, and Neolone™ 950 preservative to Part 3 with stirring. The combined mixture is Part 4.
6. Predilute Polymer #6 with a portion of the total deionized water charge. This is Part 5.
7. Gradually add Part 4 to Part 5 with stirring. Allow sample to equilibrate for > 1h.
8. The resulting hair gel had pH of 7.2 and Brookfield Viscosity of 54000 cps (Spindle RV#7, 20 rpm).

### Example 2. High Gloss, Low Viscosity Hair Gel

### Composition:

| **Ingredient** | **Weight Percent in Final Formulation** | **Trade Name (Supplier)** |
|---|---|---|
| Acrylates/Hydroxyest ers Acrylates Copolymer | 2.0 | Acudyne 180 (Rohm and Haas Company) |
| Aminomethyl Propanol | 0.42 | AMP-95 (Angus) |
| Glycerin | 2.5 | Glycerin (Merck) |
| Panthenol | 0.1 | D-Panthenol USP (Roche) |
| PEG/PPG-15/15 Dimethicone | 0.5 | DC5330 Fluid (Dow Corning) |
| EDTA | 0.05 | Titriplex III (Merck) |
| Fragrance | 0.18 | |
| Polysorbate 20 | 0.72 | Crillet I (Croda) |
| Methylisothiazolinone | 0.10 | Neolone 950 (Rohm and Haas Company) |
| Polymer #6 | 3.1 | |
| Deionized Water | 90.33 | |

### Method of preparation:

1. Predilute Acrylates/Hydroxyesters Acrylates Copolymer with a portion of the total deionized water charge. This is Part 1.
2. Predilute Aminomethyl Propanol with a portion of the total deionized water charge. This is Part 2.
3. Add Part 1 to Part 2 with stirring. The combined mixture is Part 3.
4. Combine fragrance and Polysorbate 20.
5. Add Glycerin, Panthenol, PEG/PPG-15/15 Dimethicone, EDTA, Fragrance/Polysorbate 20 mixture, and Neolone™ 950 preservative to Part 3 with stirring. The combined mixture is Part 4.
6. Predilute Polymer #6 with a portion of the total deionized water charge. This is Part 5.
7. Gradually add Part 4 to Part 5 with stirring. Allow sample to equilibrate for > 1h.
8. The resulting hair gel had pH of 7.0-7.5 and Brookfield Viscosity of 10000-13000 cps (Spindle RV#4, 10 rpm).

### Example 3: Spray Hair Gel

### Composition:

| **Ingredient** | **Weight Percent in Final Formulation** | **Trade Name (Supplier)** |
|---|---|---|
| Polymer #6 | 2.6 | |
| Oleth-20 | 0.15 | Brij™ 98 (Uniqema Americas) |
| Sorbitol | 0.5 | Sorbitol |
| Aminomethyl Propanol | 0.25 | AMP-95 (Angus) |
| PVP (10%) | 10.0 | Polyvinylpyrrolidone |
| Methylisothiazolinone | 0.10 | Neolone™ 950 preservative (Rohm and Haas Company) |
| Deionized Water | 86.4 | |

### Method of preparation:

1. Combine Polymer #6 with 85% of total Deionized Water charge with stirring. This is Part 1.
2. Slowly add Oleth-20 and Sorbitol to Part 1 with stirring. The combined mixture is Part 2.
3. Add Aminomethyl Propanol to Part 2 with stirring. The combined mixture is Part 3.
4. Add PVP solution to Part 3 with stirring. Add remaining Deionized Water and Methylisothizolinone and mix well.
5. The resulting hair gel had pH of 7.4 and Brookfield Viscosity of 9000cps (Spindle RV#4, 12 rpm).

### Example 4: Self Thickening Hair Gel

In this formulation, the polymeric rheology modifier also acts as a hair fixative.

### Composition:

| **Ingredient** | **Weight Percent in Final Formulation** | **Trade Name (Supplier)** |
|---|---|---|
| Aminomethyl Propanol | 0.7 | AMP-95 (Angus) |
| Polymer #6 | 3.5 | |
| Deionized Water | 95.8 | |

### Method of preparation:

1. Combine Polymer #6 with Deionized water with stirring.
2. Add Aminomethyl Propanol with stirring.
3. Allow sample to equilibrate for > 1h.
4. The resulting hair gel had pH of 7.0 and Brookfield Viscosity of 12150 cps (Spindle RV#4, 20 rpm).

### Example 5: Anti Dandruff Shampoo (Reference Example)

### Composition:

| **Ingredient** | **Weight Percent in Final Formulation** | **Trade Name (Supplier)** |
|---|---|---|
| Polymer #1 | 5.1 | |
| Sodium Lauryl Sulfate (30%) | 16.5 | Polystep™ B-5 (Stepan) |
| Sodium Laureth Sulfate (26%) | 15.4 | Steol™ CS-230 (Stepan) |
| Sodium Hydroxide (20%) | to pH 6.25-6.75 | |
| Cocamidopropyl Betaine (30%) | 4.6 | Amphosol™ CA (Stepan) |
| Citric Acid (50%) | to pH 5.5 | |
| Zinc Pyrithione (48%) | 2.5 | Zinc OMADINE™ biocide (Arch Chemical) |
| Dye | 0.1 | |
| Sodium Chloride | 1.5 | |
| Methylchloroisothiazolinone, Methylisothiazolinone | 0.1 | Kathon™ CG preservative(Rohm and Haas Company) |
| Deionized Water | q.s. | |

### Method of preparation:

1. Predilute Polymer #1 with 80% of the total deionized water charge. Add Sodium Lauryl Sulfate and Sodium Laureth Sulfate with stirring. The combined mixture is Part 1.
2. Adjust pH of Part 1 to pH 6.25-6.75 with 20% Sodium Hydroxide.
3. Combine 10% of the total deionized water charge with the Methylchloroisothiazolinone, methylisothiazolinone. Add this combined mixture to Part 1. This is Part 2.
4. Add Cocamidopropyl Betaine to Part 2 with stirring. The combined mixture is Part 3.
5. Adjust pH of Part 3 to pH 5.25-5.75 with 50% Citric Acid.
6. Add Zinc Pyrithione, Dye, and Sodium Chloride and stir to combine.
7. q.s. with deionized water. Allow sample to equilibrate for > 1h.
8. The resulting antidandruff shampoo had pH of 5.4 and Brookfield Viscosity of 3480 cps (Spindle RV#4, 20 rpm). The shampoo was stable (no phase separation observed) at 25 °C and 45 °C.

### Example 6. Clear Conditioning Shampoo (Reference Example)

### Composition:

| Ingredient | Weight Percent in Final Formulation | Trade Name (Supplier) |
|---|---|---|
| Polymer #6 | 3.0 | |
| Sodium Lauryl Ether Sulfate (25%) | 50.4 | Emal™ 270 N (Kao) |
| EDTA | 0.1 | Titriplex™ III (Merck) |
| Potassium Hydroxide (10%) | to pH 6.0-6.5 | |
| Cocamide DEA | 3.0 | Comperlan™ KD (Cognis) |
| Cocamidopropyl Betaine (35%) | 7.0 | Dehyton™ K (Cognis) |
| Bis (C13-15 Alkoxy) PG Amodimethicone | 1.0 | DC 8500 (Dow Corning) |
| Panthenol | 0.2 | D-Panthenol (Universal Preserv-A-Chem) |
| Polyquaternium 10 | 0.2 | Celquat™ SC240C (National Starch) |
| Polysorbate 20 | 0.22 | Tween™ 20 (ICI) |
| Fragrance | 0.22 | |
| Propylene Glycol | to adjust viscosity | Unipeg™ PG (Universal Preserv-A-Chem) |
| Methylchloroisothiazolinone, Methylisothiazolinone | 0.1 | Kathon™ CG preservative (Rohm and Haas Company) |
| Deionized Water | q.s. | |

### Method of preparation:

1. Predilute Polymer #6 with a portion of the total deionized water charge. Add Sodium Lauryl Ether Sulfate and EDTA with stirring. The combined mixture is Part 1.
2. Adjust pH of Part 1 to pH 6.0-6.5 with 10% Potassium Hydroxide.
3. Add Cocamide DEA, Cocamidopropyl Betaine, Bis (C13-15 Alkoxy) PG Amodimethicone, D-Panthenol, and Celquat™ SC240C in order into Part 1 with stirring. The combined mixture is Part 2.
4. Combine fragrance and Polysorbate 20. Add this mixture to Part 2 and stir to combine.
5. Adjust viscosity to 8000-10000 cps with Propylene Glycol.
6. Add Methylchloroisothiazolinone, methylisothiazolinone and stir to combine.
7. q.s. with deionized water.
8. The resulting clear conditioning shampoo had pH of 6.0-6.5 and Brookfield Viscosity of 8000-10000 cps (Spindle RV#4, 10 rpm).

### Example 7. 2 in 1 Conditioning Shampoo (Reference Example)

### Composition:

| **Ingredient** | **Weight Percent in Final Formulation** | **Trade Name (Supplier)** |
|---|---|---|
| Polymer #6 | 6.8 | |
| Sodium Lauryl Ether Sulfate (25%) | 50.3 | Emal™ 270 N (Kao) |
| EDTA | 0.05 | Titriplex™ III (Merck) |
| Sodium Hydroxide (20%) | to pH 5.7-6.2 | |
| Cocamidopropyl Betaine (30%) | 8.2 | Amphitol™ 55AB (Kao) |
| Glycol Distearate (and) Laureth-4 (and) Cocamidopropyl Betaine | 3.0 | Euperlan™ PK-3000 (Cognis) |
| Dimethicone (and) Laureth-23 and C12-15 Pareth-3 | 5.0 | DC 2-1491 Emulsion (Dow Corning) |
| Fragrance | 0.22 | |
| Polysorbate 20 | 0.66 | Crillet™ I (Croda) |
| Propylene Glycol | to adjust viscosity | Unipeg™ PG (Universal Preserv-A-Chem) |
| Methylchloroisothiazolinone, Methylisothiazolinone | 0.1 | Kathon™ CG preservative (Rohm and Haas Company) |
| Deionized Water | q.s. | |

### Method of preparation:

1. Predilute Polymer #6 with a portion of the total deionized water charge. Add Sodium Lauryl Ether Sulfate and EDTA with stirring. The combined mixture is Part 1.
2. Adjust pH of Part 1 to pH 5.7-6.2 with 20% Sodium Hydroxide.
3. Add Cocamidopropyl Betaine, Glycol Distearate (and) Laureth-4 (and) Cocamidopropyl Betaine, Dimethicone (and) Laureth-23 and C12-15 Pareth-3 in order into Part 1 with stirring. The combined mixture is Part 2.
4. Combine fragrance and Polysorbate 20. Add this mixture to Part 2 and stir to combine.
5. Adjust viscosity to 11000-13000 cps with Propylene Glycol.
6. Add Methylchloroisothiazolinone, methylisothiazolinone and stir to combine.
7. q.s. with deionized water
8. The resulting 2 in 1 conditioning shampoo had pH of 5.7-6.2 and Brookfield Viscosity of 11000-13000 cps (Spindle RV#4, 10 rpm). The shampoo was stable (no phase separation observed) at 25 °C and 45 °C.

### Example 8. Shower Gel with Suspended Jojoba Beads (Reference Example)

### Composition:

| **Ingredient** | **Weight Percent in Final Formulation** | **Trade Name (Supplier)** |
|---|---|---|
| Polymer #6 | 7.5 | |
| Sodium Lauryl Ether Sulfate (25%) | 50.4 | Emal™ 270 N (Kao) |
| EDTA | 0.05 | Titriplex™ III (Merck) |
| Potassium Hydroxide (10%) | to pH 6.0-6.5 | |
| Cocamidopropyl Betaine | 5.0 | Amonyl™ 380 BA (Seppic) |
| Fragrance | 0.22 | |
| Polysorbate 20 | 0.88 | Crillet™ I (Croda) |
| Propylene Glycol | to adjust viscosity | Unipeg™ PG (Universal Preserv-A-Chem) |
| Methylchloroisothiazolinone, Methylisothiazolinone | 0.1 | Kathon™ CG preservative (Rohm and Haas Company) |
| Jojoba Beads | 0.2 | Jojoba Wax Prills 40/60 (A&E Connock) |
| Deionized Water | q.s. | |

### Method of preparation:

1. Predilute Polymer #6 with a portion of the total deionized water charge. Add Sodium Lauryl Ether Sulfate and EDTA with stirring. The combined mixture is Part 1.
2. Adjust pH of Part 1 to pH 6.0-6.5 with 10% Potassium Hydroxide.
3. Add Cocamidopropyl Betaine to Part 1 with stirring. The combined mixture is Part 2.
4. Combine fragrance and Polysorbate 20. Add this mixture to Part 2 and stir to combine.
5. Adjust viscosity to 11000-13000 cps with Propylene Glycol.
6. Add Methylchloroisothiazolinone, methylisothiazolinone and stir to combine.
7. Add Jojoba Beads and stir to combine
8. q.s. with deionized water
9. The resulting shower gel had pH of 6.0-6.5 and Brookfield Viscosity of 12400 cps (Spindle RV#4, 10 rpm). The suspended bead formulation was stable at both 25 °C and 45 °C.

### Example 9. Oil in Water Emulsion (Reference Example)

### Composition:

| **Ingredient** | **Weight Percent in Final Formulation** | **Trade Name (Supplier)** |
|---|---|---|
| Polymer #6 | 6.7 | |
| Aminomethylpropanol | 0.6 | AMP-95 (Angus) |
| Methylisothiazolinone | 0.1 | Neolone™ 950 preservative (Rohm and Haas Company) |
| Paraffin Oil | 30.0 | Mineral Oil |
| Deionized Water | q.s. | |

### Method of preparation:

1. Disperse Polymer #6 and Methylisothiazolinone with the deionized water.
2. Adjust pH to 6.1 with Aminomethylpropanol.
3. Add paraffin oil using a high shear homogenizer at 10,000 rpm for 5 minutes to form emulsion.
4. The resulting emulsion had pH of 6.1.

### Example 10. Waterproof Sunscreen (Reference Example)

A typical waterproof sunscreen formulation using the crosslinked copolymers described herein is as follows. Addition of the copolymer to the formulation would allow for effective thickening of the sunscreen and would provide enhanced film formulation and good feel on the skin. Typically, up to 10% of SunSpheres™ (Styrene/Acrylates Copolymer) SPF Enhancer, Rohm and Haas Company) may be added to the formulation for SPF enhancement. Typical Composition:

| **Ingredient** | **Weight Percent in Final Formulation** | **Trade Name (Supplier)** |
|---|---|---|
| Deionized Water | 64.25 | |
| Crosslinked Copolymer | 2.0 | |
| Acrylates Copolymer (28%) | 2.0 | Aculyn™ 33 rheology modifier (Rohm and Haas Company) |
| Propylene Glycol | 1.0 | Propylene Glycol (BASF) |
| Isopropyl Myristate | 5.0 | Crodamol™ IPM (Croda) |
| Cyclomethicone | 1.0 | Dow Corning 344 Fluid (Dow Corning) |
| Cetearyl Alcohol | 1.0 | Lanette™ O (Cognis) |
| DEA Cetyl Alcohol | 4.0 | Crodafos™ CDP (Croda) |
| Benzophenone-3 | 6.0 | Neo Heliopan™, Type BB (Haarmann & Reimer) |
| Ethylhexyl Methoxycinnamate | 7.5 | Neo Heliopan™, Type AV (Haarmann & Reimer) |
| Macadamia Ternifolia Seed Oil | 5.0 | Cropure™ Macadamian (Croda) |
| Tocopheryl Acetate | 0.05 | Copherol™ 1250 (Cognis) |
| Methylisothiazolinone (9.5%) | 0.1 | Neolone™ 950 preservative (Rohm and Haas Company) |
| Styrene/Acrylates Copolymer | 0.1 | Acusol™ OP301 Emulsion (Rohm and Haas Company) |
| Fragrance and Dye | 1.0 | |

### Typical Method of preparation:

1. Combine Water, Crosslinked Copolymer, Acrylates Copolymer, and Propylene Glycol with stirring. Heat mixture to 75 °C. This is Part 1.
2. In a second vessel, combine Isopropyl Myristate, Cyclomethicone, Cetearyl Alcohol, DEA Cetyl Alcohol, Benzophenone-3, Ethylhexyl Methoxycinnamate, Macadamia Ternifolia Seed Oil, and Tocopheryl Acetate. Heat mixture to 75 °C. This is Part 2.
3. Slowly add Part 2 to Part 1 with stirring, avoiding air entrapment.
4. Cool to 35 °C with stirring and add Methylisothiazolinone, Styrene/Acrylates Copolymer, Fragrance, and Dye.

### Example 11. Two Part Oxidative Hair Dye (Reference Example)

A typical two part oxidative hair dye formulation using the crosslinked copolymers described herein is as follows. Addition of the copolymer to the formulation would allow for rapid thickening of the hair dye upon combination, allowing for preparation of a no-run hair dye to provide effective coverage on the hair.

### Typical Composition:

| **Ingredient** | **Weight Percent in Final Formulation** | **Trade Name (Supplier)** |
|---|---|---|
| **PART A (Dye Base)** | | |
| Deionized Water | 87.56-89.56 | |
| Dye Mix | 0.5-2.5 | (H. Lowenstein) |
| Sodium Sulfite | 0.3 | Sodium Sulfite (Spectrum) |
| Disodium EDTA | 0.1 | Versene™ NA (Dow Chemical) |
| Decyl Glucoside | 0.5 | Plantaren™ 2000 (Cognis) |
| Ethanolamine | 4.0 | Mealan™ (R.I.T.A.) |
| Cocamidopropyl betaine (30%) | 5.0 | Amphosol™ CA (Stepan) |
| Methylisothiazolinone | 0.08 | Neolone™ 950 preservative (Rohm and Haas Company) |

| **PART B (Developer)** | | |
|---|---|---|
| Deionized Water | 62.9 | |
| Crosslinked Copolymer | 5.0 | |
| C14-15 pareth-7 | 10.0 | Neodol™ 45-7 (Shell Chemical) |
| C12-15 pareth-3 | 10.0 | Neodol™ 25-3 (Shell Chemical) |
| Hydrogen Peroxide (50%) | 12.0 | Super D H₂O₂ (FMC) |
| Etidronic Acid | 0.1 | Etidronic Acid (Sabo) |

### Typical Method of preparation:

### PART A:

1. Heat the deionized water to 45 °C. Add the dye mixture to the water with stirring.
2. To the diluted dye mixture, add sodium sulfite, Disodium EDTA, Decyl glucoside, Ethanolamine and Cocamidopropyl betaine with stirring.
3. Cool to ambient temperature and add Methylisothiazolinone.

### PART B:

1. Combine the Crosslinked Copolymer and deionized water in a second vessel.
2. Add the C14-15 pareth-7 to the diluted rheology modifier mixture and stir to combine.
3. Upon dissolution of the C14-15 pareth-7, add the C12-15 pareth-3 and stir to combine.
4. With constant agitation, add the Hydrogen Peroxide.
5. Add the etidronic acid with stirring.

### Example 12: Salicylic Acid Facial Scrub (Reference Example)

A facial scrub formulation containing salicylic acid using the crosslinked copolymers described herein is as follows. Addition of the copolymer to the formulation would allow preparation of smooth flowing formulation and allow for stabilization of bead suspension.

### Typical Composition:

| **Ingredient** | **Weight Percent in Final Formulation** | **Trade Name (Supplier)** |
|---|---|---|
| Crosslinked Copolymer | 5.0 | |
| Sodium Olefin Sulfonate (40%) | 25.0 | Bioterge™ AS-40 (Stepan) |
| Sodium Hydroxide (20%) | To pH 5.3 | |
| Glycerin | 2.0 | |
| Salicylic Acid | 2.0 | |
| Cocamidopropyl Betaine (30%) | 10.0 | Amphosol™ CA (Stepan) |
| Potassium C12-13 Alkyl Phosphate (40%) | 2.0 | Arlatone™ MAP 230-K40 (Uniqema Americas) |
| Dye | 0.2 | |
| Jojoba Beads | 2.0 | Jojoba Wax Prills 40/60 (A&E Connock) |
| Deionized Water | q.s. | |

### Typical Method of preparation:

1. Predilute Crosslinked Copolymer with 65% of the total deionized water charge. Add 60% of the total Sodium Olefin Sulfonate charge with stirring. The combined mixture is Part 1.
2. Adjust pH of Part 1 to pH 5.1-5.5 with 20% Sodium Hydroxide.
3. In a second vessel, combine 25% of the total deionized water charge, 40% of the total Sodium Olefin Sulfonate charge, Glycerin, and Salicylic Acid with stirring. This is Part 2.
4. Slowly combine Parts 1 and 2 with stirring. The combined mixture is Part 3.
5. Add Cocamidopropyl Betaine, Potassium C12-13 Alkyl Phosphate, Dye, and Jojoba Beads to Part 3 with stirring. Q.s. with Deionized Water.

### Example 13: Soap Based Facial Cleanser (Reference Example)

A typical soap based facial cleanser using the crosslinked copolymers described herein is as follows. Addition of the copolymer to the formulation would allow for effective thickening and preparation of a stable facial cleanser with target viscosity of 50000-100000cps.

### Typical Composition:

| **Ingredient** | **Weight Percent in Final Formulation** | **Trade Name (Supplier)** |
|---|---|---|
| Potassium Hydroxide | 5.0 | |
| Deionized Water | q.s. | |
| Lauric Acid | 10.5 | Kortacid™ 1299 (Akzonobel) |
| Myristic Acid | 7.0 | Kortacid™ 1499 (Akzonobel) |
| Crosslinked Copolymer | 4.0 | |
| Potassium Hydroxide (10%) | To target pH | |
| Sodium Lauroyl Sarcosinate | 8.0 | Secosyl™ (Stepan) |
| Lauramine Oxide | 3.0 | Ammonyx™ LO (Stepan) |
| Glycerin | 6.0 | Glycerin (Merck) |
| EDTA | 0.05 | Titriplex™ III (Merck) |
| Styrene/Acrylamide Copolymer | 1.0 | Acusol™ OP303P Opacifier (Rohm and Haas Company) |
| Fragrance | 0.3 | |
| Polysorbate 20 | 1.2 | Crillet™ I (Croda) |
| Methylisothiazolinone | 0.1 | Neolone™ 950 preservative (Rohm and Haas Company) |

### Typical Method of preparation:

1. Dissolve Potassium Hydroxide in 16% of the total deionized water. Upon dissolution, add an additional portion of deionized water (38% of total charge) previously heated to 60 °C. This is Part 1.
2. Combine Lauric Acid and Myristic Acid with stirring and heat to 60-65 °C. This is Part 2. Add Part 2 into Part 1 with stirring. Maintain the temperature of the mixture at 60 °C. The combined mixture is Part 3.
3. Dilute the Crosslinked Copolymer with 25% of the total deionized water. Adjust the pH of this mixture to pH 5.5 with 10% Potassium Hydroxide solution. Add to Part 3 with stirring. The combined mixture is Part 4.
4. Combine fragrance and Polysorbate 20.
5. Combine 12% of the total deionized water, Sodium Lauroyl Sarcosinate, Lauramine Oxide, Glycerin, and EDTA with stirring. Add to Part 4 with stirring. The combined mixture is Part 5.
6. Combine fragrance and Polysorbate 20. Add fragrance/Polysorbate 20 and Methylisothiazolinone to Part 5 with stirring. The combined mixture is Part 6.
   Dilute Styrene/Acrylamide Copolymer with 5% of the total deionized water charge. Add to Part 6 with stirring.
7. Add 10% Potassium Hydroxide solution as needed to adjust final pH of the mixture to pH 8.5-9.0.
8. If necessary, add 25% NaCl solution with stirring to adjust final viscosity of mixture. Target viscosity = 50000-100000 cps.

### Example 14: Soap Based Body Wash (Reference Example)

A typical soap based body wash formulation using the crosslinked copolymers described herein is as follows. Addition of the copolymer to the formulation would allow for effective thickening and preparation of a stable body wash.

### Typical Composition:

| **Ingredient** | **Weight Percent in Final Formulation** | **Trade Name (Supplier)** |
|---|---|---|
| Potassium Hydroxide | 5.36 | |
| Deionized Water | q.s. | |
| Lauric Acid | 10.0 | Kortacid™ 1299 (Akzonobel) |
| Myristic Acid | 7.2 | Kortacid™ 1499 (Akzonobel) |
| Palmitic Acid | 1.3 | Kortacid™ 1695 (Akzonobel) |
| Crosslinked | 3.0 | |
| Copolymer | | |
| Potassium Hydroxide (10%) | To target pH | |
| Sodium Lauryl Ether Sulfate *(25%)* | 5.0 | Texapon™ N70 RI (Cognis)* **Dilute Texapon N70 RI from 70% to 25% prior to use in formulation* |
| Propylene Glycol | 1.0 | Propylene glycol (Fluka) |
| Decyl Glucoside (50%) | 3.0 | Oramix™ NS 10 (Seppic) |
| Styrene/Acrylamide Copolymer | 0.8 | Acusol™ OP303P Opacifier (Rohm and Haas Company) |
| Sodium Chloride (25%) | To target viscosity | |

### Typical Method of preparation:

1. Dissolve Potassium Hydroxide in 16% of the total deionized water. Upon dissolution, add an additional portion of deionized water (38% of total charge) previously heated to 60 °C. This is Part 1.
2. Combine Lauric Acid, Myristic Acid, and Palmitic Acid with stirring and heat to 60-65 °C. This is Part 2.
3. Add Part 2 into Part 1 with stirring. Maintain the temperature of the mixture at 60 °C. The combined mixture is Part 3.
4. Dilute the Crosslinked Copolymer with 25% of the total deionized water. Adjust the pH of this mixture to pH 5.5 with 10% Potassium Hydroxide solution. Add to Part 3 with stirring. The combined mixture is Part 4.
5. Combine 12% of the total deionized water, Sodium Lauryl Ether Sulfate, Propylene Glycol, and Decyl Glucoside with stirring. Add to Part 4 with stirring. The combined mixture is Part 5.
6. Dilute Styrene/Acrylamide Copolymer with the 5% of the total deionized water charge. Add to Part 5 with stirring.
   Add 10% Potassium Hydroxide solution as needed to adjust final pH of the mixture to pH 9.0-9.5.
7. Add 25% NaCl solution with stirring to adjust final viscosity of mixture. Q.s. with deionized water. Target viscosity = 1000 cps.

## Claims

1. The use in a hair gel of an aqueous composition that when applied to a substrate the composition smoothly spreads to form a continuous film without breaking up, forming lumps or rough sheets, the composition comprising
a) from 0.1 to 8 weight percent of one or more crosslinked copolymers, wherein:
each of the one or more crosslinked copolymers independently comprises from 2.5 to 35 weight percent (meth)acrylic acid residues, from 10 to 80 weight percent C2-C4 alkyl (meth)acrylate residues, from 2 to 25 weight percent lipophilically modified (meth)acrylate residues, and from 0.001 to 7.5 weight percent residues of a crosslinker wherein the crosslinker has no ester or amide functionality; and
b) the pH of the composition is from 4 to 10.

2. The use of claim 1 in which the crosslinker is one or more of divinylbenzene, trimethylolpropane diallyl ether, and tetraallyl pentaerythritol.

3. The use of claim 1 in which one or more of the crosslinked copolymers comprises from 5 to 25 weight percent acrylic acid residues.

4. The use of claim 1 comprising from 0.75 to 4 weight percent of one or more of the crosslinked copolymers.

5. The use of claim 1 in which at least one of the crosslinked copolymers comprises methacrylic acid residues and acrylic acid residues, and wherein the sum of the acrylic acid plus the methacrylic acid residues total from 20 to 40 weight percent of the copolymer.

6. The use of claim 1 wherein the crosslinked copolymer comprises from 0.1 to 2.5 weight percent crosslinker.

7. The use of claim 1 in which the crosslinked copolymer contains from 16 to 20 weight percent lipophilically modified (meth)acrylate residues.

8. The use of claim 1, wherein the gel further comprises one or more additional rheology modifiers.

9. The use of claim 8 wherein at least one of the additional rheology modifiers is an Acrylates Copolymer or PEG-150 distearate.

## Patentansprüche

1. Verwendung von einer wässrigen Zusammensetzung in einem Haargel, wobei sich die Zusammensetzung, wenn sie auf einem Substrat aufgebracht wird, problemlos ausbreitet, um einen kontinuierlichen Film zu bilden, ohne aufzubrechen, Klumpen oder rauhe Schichten zu bilden, wobei die Zusammensetzung umfasst
a) von 0,1 bis 8 Gewichtsprozent von einem oder mehreren vernetzten Copolymer(en), wobei:
jedes von dem einen oder mehreren vernetzten Copolymer(en) unabhängig von 2,5 bis 35 Gewichtsprozent (Meth)acrylsäurereste, von 10 bis 80 Gewichtsprozent C2-C4 Alkyl(meth)acrylatreste, von 2 bis 25 Gewichtsprozent lipophilisch-modifizierte (Meth)acrylatreste, und von 0,001 bis 7,5 Gewichtsprozent Reste von einem Vernetzer umfasst, wobei der Vernetzer keine Ester- oder Amidfunktionalität aufweist; und
b) der pH der Zusammensetzung von 4 bis 10 beträgt.

2. Verwendung nach Anspruch 1, in welcher der Vernetzer einer oder mehrere aus Divinylbenzol, Trimethylolpropandiallylether und Tetraallylpentaerythritol ist.

3. Verwendung nach Anspruch 1, in welcher eines oder mehrere der vernetzten Copolymeren von 5 bis 25 Gewichtsprozent Acrylsäurereste umfasst bzw. umfassen.

4. Verwendung nach Anspruch 1, umfassend von 0,75 bis 4 Gewichtsprozent von einem oder mehreren der vernetzten Copolymeren.

5. Verwendung nach Anspruch 1, in welcher mindestens eines der vernetzten Copolymere Methacrylsäurereste und Acrylsäurereste umfasst, und wobei die Summe aus den Acrylsäure- plus den Methacrylsäureresten auf 20 bis 40 Gewichtsprozent des Copolymers beträgt.

6. Verwendung nach Anspruch 1, wobei das vernetzte Copolymer von 0,1 bis 2,5 Gewichtsprozent Vernetzer umfasst.

7. Verwendung nach Anspruch 1, in welcher das vernetzte Copolymer von 16 bis 20 Gewichtsprozent lipophilisch-modifizierte (Meth)acrylatreste enthält.

8. Verwendung nach Anspruch 1, wobei das Gel weiter einen oder mehrere zusätzliche(n) Rheologiemodifizierer umfasst.

9. Verwendung nach Anspruch 8, wobei mindestens einer der zusätzlichen Rheologiemodifizierer ein Copolymer aus Acrylaten oder PEG-150-Distearat ist.

## Revendications

1. Utilisation dans un gel capillaire d'une composition aqueuse dans laquelle, lorsqu'elle est appliquée sur un substrat, la composition s'étale régulièrement pour former un film continu sans rupture, formation de grumeaux ou de feuilles rugueuses, la composition comprenant
a) de 0,1 à 8 % en poids d'un ou plusieurs copolymères réticulés, où :
chacun des un ou plusieurs copolymères réticulés comprend indépendamment de 2,5 à 35 % en poids de résidus d'acide (méth)acrylique, de 10 à 80 % en poids de résidus de (méth)acrylate d'alkyle en C2-C4, de 2 à 25 % en poids de résidus de (méth)acrylate modifiés par voie lipophile et de 0,001 à 7,5 % en poids de résidus d'un agent de réticulation, où l'agent de réticulation ne présente pas de fonctionnalité ester, ni amide ; et
b) le pH de la composition est de 4 à 10.

2. Utilisation selon la revendication 1, dans laquelle l'agent de réticulation est un ou plusieurs parmi le divinylbenzène, le diallyléther de triméthylolpropane et le tétraallylpentaérythritol.

3. Utilisation selon la revendication 1, dans laquelle un ou plusieurs des copolymères réticulés comprend de 5 à 25 % en poids de résidus d'acide acrylique.

4. Utilisation selon la revendication 1 comprenant de 0,75 à 4 % en poids d'un ou plusieurs des copolymères réticulés.

5. Utilisation selon la revendication 1, dans laquelle au moins un des copolymères réticulés comprend des résidus d'acide méthacrylique et des résidus d'acide acrylique et dans laquelle la somme des résidus d'acide acrylique plus d'acide méthacrylique totalise de 20 à 40 % en poids du copolymère.

6. Utilisation selon la revendication 1, dans laquelle le copolymère réticulé comprend de 0,1 à 2,5 % en poids d'agent de réticulation.

7. Utilisation selon la revendication 1, dans laquelle le copolymère réticulé contient de 16 à 20 % en poids de résidus de (méth)acrylate modifiés par voie lipophile.

8. Utilisation selon la revendication 1, dans laquelle le gel comprend de plus un ou plusieurs agents de modification de la rhéologie supplémentaires.

9. Utilisation selon la revendication 8, dans laquelle au moins un des agents de modification de la rhéologie supplémentaires est un copolymère d'acrylate ou le distéarate de PEG-150.
